# DEMANDE DE BREVET EUROPEEN

(11) **EP 1 000 608 A1**
(43) Date de publication de la demande: **17.05.2000**
(21) Numéro de dépôt: 99402408.1
(22) Date de dépôt: 01.10.1999
(51) Int. Cl.: A61K 7/035, A61K 7/48

(54) **Poudre cosmétique et/ou dermatologique, son procédé de préparation et ses utilisations**

(30) Priorité: 06.11.1998 FR 9814029
(71) Demandeur: L'OREAL, 75008 Paris (FR)
(72) Inventeur: Demars, Gwennaelle, 92340 Bourg La Reine (FR); Koely, Sandrine, 92160 Antony (FR)
(74) Mandataire: Rasson, Catherine

(57) **Abrégé**

La présente invention se rapporte à une poudre cosmétique et/ou dermatologique caractérisée en ce qu'elle comprend (i) de 5 à 60 % en poids, par rapport au poids total de la poudre, d'au moins un amidon modifié, (ii) de 30 à 90 % en poids, par rapport au poids total de la poudre, d'une phase huileuse comprenant au moins une huile, et (iii) au moins un électrolyte, le rapport pondéral phase huileuse/amidon étant égal ou supérieur à 1.

L'invention a encore pour objet un procédé de fabrication de ladite poudre, comprenant (1) la préparation d'une dispersion huile-dans-eau par mélange d'une phase huileuse comprenant au moins une huile dans une phase aqueuse comprenant au moins un amidon modifié et un électrolyte, le rapport pondéral phase huileuse/amidon étant égal ou supérieur à 1, et (2) la déshydratation de ladite dispersion pour obtenir ladite poudre.

La poudre selon l'invention a l'avantage de pouvoir contenir une quantité importante d'électrolytes. Elle peut être utilisée telle quelle ou incorporée dans une composition cosmétique et/ou dermatologique. La poudre ou la composition la contenant sont utiles notamment pour le traitement et le soin de la peau, des muqueuses, du cuir chevelu et/ou des cheveux, en particulier pour le traitement de la peau sensible et du cuir chevelu sensible et/ou pour hydrater la peau. Elles peuvent être utilisées aussi pour le traitement des désordres cutanés tels que la peau sèche, le psoriasis, les dermites atopiques, le vitiligo et le mycosis fongoïde.

## Description

L'invention se rapporte à une poudre cosmétique ou dermatologique contenant au moins un électrolyte, à son procédé de fabrication et à une composition la contenant. L'invention a encore pour objet les utilisations de ladite poudre ou de ladite composition notamment pour le soin et/ou le traitement de la peau, des muqueuses et/ou du cuir chevelu, ainsi que pour le traitement des désordres cutanés.

On connaît dans le domaine cosmétique ou dermatologique, des compositions contenant des électrolytes (sels inorganiques et organiques) utilisés pour leurs effets bénéfiques sur la peau. Certains électrolytes permettent notamment de traiter les problèmes de peau sensible et les désordres pathologiques ou physiologiques associés à la libération de la substance P et/ou du TNF-alpha (Tumor Necrosis Factor-alpha) et notamment de traiter les peaux sensibles, les désordres et maladies cutanées. De telles compositions sont décrites par exemple dans les documents EP-A-737471, EP-A-770392 et EP-A-775492.

Par ailleurs, il est connu que l'incorporation d'électrolytes dans les compositions cosmétiques ou dermatologiques contenant de l'eau posent des problèmes du fait de leur incompatibilité avec certains constituants habituellement utilisés dans de telles compositions. Ainsi par exemple les électrolytes sont incompatibles avec les polymères carboxyvinyliques neutralisés habituellement utilisés comme gélifiants, du fait qu'ils "cassent" l'émulsion et la liquéfient. De ce fait, des compositions contenant des polymères carboxyvinyliques et des électrolytes sont dépourvues de consistance, ce qui va à l'encontre du résultat recherché par l'emploi d'un gélifiant.

La demanderesse a trouvé de manière inattendue qu'il était possible d'obtenir une composition dépourvue des inconvénients des compositions de l'art antérieur par l'incorporation d'électrolytes dans une poudre anhydre, contenant un amidon modifié et pouvant contenir au moins un corps gras.

Certes, le document JP-A-7/206663 décrit une poudre pour le bain, contenant des sels minéraux, des sucres et des corps gras. Toutefois, cette poudre présente l'inconvénient d'être sensible à l'humidité et à la contamination bactériologique.

La poudre selon l'invention n'a pas ces inconvénients.

La présente invention a donc pour objet une poudre cosmétique et/ou dermatologique, caractérisée en ce qu'elle comprend (i) de 5 à 60 % en poids, par rapport au poids total de la poudre, d'au moins un amidon modifié par au moins une réaction choisie parmi la prégélatinisation, l'oxydation, la réticulation et l'estérification, (ii) de 30 à 90 % en poids, par rapport au poids total de la poudre, d'une phase huileuse comprenant au moins une huile, et (iii) au moins un électrolyte, le rapport pondéral phase huileuse/amidon étant égal ou supérieur à 1.

On entend par "poudre" une substance solide divisée en particules ou grains très fins et homogènes.

La poudre selon l'invention est de préférence exempte de protéine.

Selon un mode particulier de réalisation de l'invention, la poudre comprend de 5 à 50 % en poids d'au moins un amidon modifié, de 0,1 à 40 % en poids d'au moins un électrolyte et de 50 à 90 % en poids d'une phase huileuse comprenant au moins une huile, par rapport au poids total de la poudre.

La poudre selon l'invention comprend une phase huileuse fixée dans l'amidon modifié.

Cette poudre présente notamment les avantages de pouvoir contenir une quantité importante d'électrolytes, d'être applicable sur tout type de peaux sans laisser d'effet gras malgré la quantité importante d'huile, d'être facile et rapide à utiliser, de pénétrer dans la peau sans laisser de film visible, de conserver les propriétés hydratantes ou nourrissantes des corps gras et de ne pas nécessiter l'ajout d'un liquide tel que l'eau puisqu'elle peut être utilisée telle quelle. En outre, on peut facilement en prélever la quantité souhaitée.

Par ailleurs, la poudre selon l'invention n'est pas sensible à l'humidité ni à la contamination bactériologique. En outre, du fait que cette poudre peut être obtenue sans émulsionnant et qu'elle se conserve bien, on peut éviter l'ajout d'émulsionnants et/ou de conservateurs, et ainsi obtenir une poudre beaucoup moins irritante que les produits de soin classiques.

En outre, une telle présentation d'un produit cosmétique permet l'incorporation de composés ayant différentes propriétés physico-chimiques. Ce produit peut ainsi comprendre des détergents. Ceci permet notamment de nettoyer la peau tout en l'hydratant et la nourrissant. On a donc à faire à un nouveau concept de produit cosmétique ou dermatologique d'applications variées, non spécifique à un type de peau.

Un autre avantage de la poudre selon l'invention est de pouvoir y incorporer des composés connus pour être sensibles à l'eau et/ou à l'oxydation tels que des vitamines (vitamine C, vitamine A ou leurs esters) ou des enzymes. Dans ce milieu, ces composés vont se conserver longtemps et donc ne pas perdre leur activité au cours du temps.

Enfin, la poudre de l'invention a encore l'avantage de pouvoir être aussi incorporée dans une composition cosmétique et/ou dermatologique, telle qu'une lotion ou une émulsion E/H ou H/E ou une émulsion triple. On obtient ainsi une composition qui, bien que contenant une forte proportion d'électrolytes, reste stable même en présence de composés avec lesquels les électrolytes sont habituellement incompatibles.

La présente invention a donc aussi pour objet une composition cosmétique et/ou dermatologique, caractérisée en ce qu'elle comprend au moins une poudre telle que définie ci-dessus.

La poudre selon l'invention présente une granulométrie (ou dimension moyenne en nombre de particules) pouvant aller notamment d'environ 0,1 à 100 µm, de préférence de 0,5 à 50 µm et mieux de 1 à 10 µm, cette granulométrie étant mesurée avec l'appareil "MICROTRAC X100 & SRA 150" de la société LEEDSNORTHRUP.

Comme électrolyte utilisable dans la composition selon l'invention, on peut citer notamment les sels des métaux mono-, di- ou trivalents, et plus particulièrement les sels de métal alcalino-terreux et en particulier les sels de baryum, de calcium et de strontium, les sels de métal alcalin et par exemple les sels de sodium et de potassium, ainsi que les sels de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.

Les ions constituant ces sels peuvent être choisis par exemple parmi les carbonates, les bicarbonates, les sulfates, les glycérophosphates, les borates, les chlorures, les nitrates, les acétates, les hydroxydes, les persulfates ainsi que les sels d'a-hydroxyacides (citrates, tartrates, lactates, malates) ou d'acides de fruits, ou encore les sels d'acides aminés (aspartate, arginate, glycocholate, fumarate).

De préférence, le sel est choisi parmi les sels de calcium, de magnésium, de sodium, de potassium et leurs mélanges, et plus particulièrement le chlorure de magnésium, le chlorure de potassium, le chlorure de sodium, le chlorure de calcium, le bromure de magnésium et leurs mélanges. Il peut s'agir en particulier d'un mélange comprenant environ de 6 à 10 % de magnésium, de 11 à 15 % de potassium, de 1 à 5 % de sodium, jusqu'à 0,5 % de calcium, de 30 à 45 % de chlorures et moins de 0,1 % de sulfates, le reste étant des insolubles et de l'eau de recristallisation qui représente environ de 27 à 40 % du mélange, le dit mélange étant ici appelé "sels de la mer morte" car il correspond à la composition des sels présents dans l'eau de la mer morte.

La quantité d'électrolyte(s) dans la poudre de l'invention peut varier dans une large mesure selon le but recherché. Cette quantité peut aller par exemple de 0,1 à 40 % et de préférence de 5 à 25 % en poids par rapport au poids total de la poudre.

L'amidon modifié utilisé dans la poudre de l'invention peut être modifié par une ou plusieurs des réactions suivantes : prégélatinisation, oxydation, réticulation, estérification.

De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymèrisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glyceryl et/ou phosphate) ;
- estérification en milieu alcalin pour le greffage de groupes fonctionnels, notamment acétyl, hydroxyéthyl, hydroxypropyl, carboxyméthyl, octénylsuccinique.

Comme amidons modifiés utilisables selon l'invention, on peut citer par exemple les amidons estérifiés par l'anhydride octénylsuccinique et plus particulièrement le "Aluminium Starch octenyl succinate" tel que le produit vendu par la société NATIONAL STARCH sous le nom de DRY-FLO, l'amidon de maïs réticulé vendu sous le nom RESISTAMYL E2 par la société AMYLUM ; l'amidon de pomme de terre estérifié par un groupe carboxyméthyl vendu sous le nom SUPRAMYL P 60 par la société AMYLUM, l'amidon de maïs estérifié par un groupe hydroxypropyle vendu sous le nom MERIGEL EF6 par la société AMYLUM ; l'amidon prégélatimisé, modifié par l'anhydride octénylsuccinique, puis par un motif hydrophobe, vendu sous le nom de NATROSORB HFB par la société NATIONAL STARCH ; l'amidon de maïs réticulé et acétylé vendu par la société CERESTAR sous le nom C* Flo 06205.

Selon un mode préféré de réalisation de l'invention, les amidons utilisés sont le DRY-FLO et le C* Flo 06205.

La phase huileuse contient au moins une huile. L'huile utilisée peut être choisie parmi les huiles minérales telles que les huiles de paraffine ou la vaseline, les huiles de silicone telles que les huiles de silicone volatiles, les huiles d'origine végétale (par exemple l'huile d'amandes douces, l'huile d'amandes d'abricot), les huiles d'origine animale, les huiles de synthèse et leurs mélanges. Le ou les huiles représentent la plus grande proportion de la phase huileuse, c'est-à-dire de 50 à 100 % en poids et de préférence de 80 à 100 % en poids de la phase huileuse.

On peut ajouter à l'huile ou aux huiles, des matières grasses telles que les acides gras, les alcools gras, les cires telles que les cires d'origine animale comme la cire d'abeille, les cires de carnauba ou de candellila, les cires minérales comme les cires microcristallines et les cires de synthèse comme les cires de polyéthylène ou de silicone.

La phase huileuse représente de 30 à 90 % en poids et de préférence de 50 à 90 % en poids par rapport au poids total de la poudre.

La poudre de l'invention peut comprendre aussi un ou plusieurs additifs couramment utilisés dans les domaines cosmétique et dermatologique. Comme additifs, on peut citer par exemple les actifs cosmétiques ou dermatologiques, les émulsionnants ou tensioactifs, les détergents, les matières colorantes y compris les pigments, les abrasifs, les agents antioxydants ou anti-radicaux libres, les charges, les parfums. Ces additifs peuvent représenter de 0 à 30 % du poids total de la poudre, de préférence de 0,5 à 15 % du poids total de la poudre.

Comme actifs, on peut citer par exemple les agents anti-acné, antimicrobiens, antitranspirants, astringents, déodorants, dépilatoires, analgésiques externes, les agents de conditionnement des cheveux, de conditionnement de la peau, de protection solaire, les vitamines, les acides gras essentiels, les agents kératolytiques, les enzymes, les agents hydratants, les anti-inflammatoires, les détergents ou agents moussants, les parfums, les charges matifiantes minérales ou organiques, les dépigmentants.

De façon avantageuse, la poudre de l'invention comporte un milieu physiologiquement acceptable pour la peau, les muqueuses et/ou les fibres kératiniques (cheveux et cils).

Un autre objet de l'invention concerne un procédé de fabrication d'une poudre, notamment cosmétique ou dermatologique, comprenant au moins un amidon modifié, une phase huileuse et un électrolyte, le dit procédé comprenant (1) la préparation d'une dispersion huile-dans-eau par mélange d'une phase huileuse comprenant au moins une huile dans une phase aqueuse comprenant au moins un amidon modifié et au moins un électrolyte, le rapport pondéral phase huileuse/amidon étant égal ou supérieur à 1, et (2) la déshydratation de ladite dispersion pour obtenir ladite poudre.

Par dispersion, on entend toute dispersion ou émulsion huile-dans-eau, c'est-à-dire tout mélange d'une phase huileuse dans une phase aqueuse en présence ou non d'un émulsionnant.

La phase aqueuse de la dispersion huile-dans-l'eau représente de préférence au moins 30 % en poids de la dispersion. La phase aqueuse comprend généralement de l'eau déminéralisée. Toutefois, dans le cadre de la présente invention, une partie ou la totalité de cette eau peut être remplacée par une eau thermale ou minérale. En général, une eau minérale est propre à la consommation, ce qui n'est pas toujours le cas d'une eau thermale. Chacune de ces eaux contient, entre autre, des minéraux solubilisés et des oligoéléments. Comme eau thermale ou minérale susceptible d'être utilisée, on peut citer par exemple l'eau de Vittel, les eaux du bassin de Vichy, l'eau d'Uriage, l'eau de La Roche Posay, l'eau de La Bourboule, l'eau d'Enghien-les-bains, l'eau de Saint-Gervais-les bains, l'eau de Néris-les-Bains, l'eau d'Allevard-les-bains, l'eau de Digne, l'eau des Maizières, l'eau de Neyrac-les-bains, l'eau de Lons le Saunier, l'eau des Eaux Bonnes, l'eau de Rochefort, l'eau de Saint Christau, l'eau des Fumades et l'eau de Tercis-les-bains.

Le rapport pondéral phase huileuse/amidon est égal ou supérieur à 1, et va de préférence de 1 à 19 et mieux de 2 à 10.

Selon un mode particulier de réalisation du procédé, la dispersion utilisée est telle qu'elle a une teneur en matière sèche allant de 5 à 70 % en poids et de préférence de 10 à 60 % en poids. Une telle teneur en matière sèche permet d'avoir une viscosité de la dispersion, telle qu'elle soit suffisamment fluide pour pouvoir être utilisée pour la suite des étapes.

La phase aqueuse peut être préparée à toute température (0 à 100°C). On travaille de préférence à une température allant de 80° à 100°C.

De manière parallèle, on prépare la phase huileuse et on l'ajoute à la phase aqueuse qui est de préférence refroidie à une température inférieure à 80°C, de préférence en une quantité telle que la teneur en matière sèche dans la dispersion qui en résulte aille de 5 à 70 % en poids par rapport au poids total de la dispersion.

On forme alors la dispersion en mélangeant lentement les phases aqueuse et huileuse, par exemple en incorporant doucement la phase huileuse dans la phase aqueuse tout en maintenant une agitation constante.

On obtient ainsi une dispersion huile-dans-eau, présentant un pH dépendant de sa composition, mais généralement compris entre 4 et 9.

De manière préférée, le procédé comprend une étape d'homogénéisation entre la préparation de la dispersion par mélange de la phase huileuse et de la phase aqueuse et avant la déshydratation de la dispersion obtenue. L'homogénéisation est avantageusement réalisée sous pression élevée, de manière à diminuer la taille moyenne des gouttelettes de la phase huileuse jusqu'à environ 350 nm, voire moins. L'homogénéisation est réalisée sous une pression en général comprise entre 300 et 600 bars, de préférence d'environ 600 bars (60 x 10⁶ Pa), pour obtenir des gouttelettes de phase huileuse ayant une taille moyenne (en nombre) de préférence inférieure à 350 nm, allant généralement de 80 à 300 nm.

La dispersion ainsi homogénéisée est alors déshydratée par tout procédé connu, et notamment par atomisation ou par lyophilisation. Selon un mode préféré de réalisation de l'invention, la déshydratation est effectuée par atomisation. Dans ce cas, la température de l'air chaud utilisé pour le séchage va de préférence d'environ 100°C à 220°C, et la température de sortie de la poudre va de préférence d'environ 30°C à 140°C. Le temps d'atomisation est très bref ; il est de préférence inférieur ou égal à 2 minutes.

La présente invention a aussi pour objet une poudre notamment cosmétique et/ou dermatologique susceptible d'être obtenue selon le procédé décrit ci-dessus.

La poudre selon l'invention peut être compactée pour gagner du volume et pour en faciliter le conditionnement, la conservation, le stockage et l'emploi. Avant le compactage éventuel de la poudre, celle-ci peut subir une étape de granulation supplémentaire ayant pour but d'homogénéiser la granulométrie de la poudre.

La poudre selon l'invention présente l'avantage d'être très stable et de se conserver plusieurs mois sans que l'on puisse observer de séparation de phases, d'évolution de la couleur, de reprise d'eau ou d'autres dégradations, microbiologiques par exemple.

Quand on l'incorpore dans une composition, celle-ci comporte de préférence une phase aqueuse. Ce peut être par exemple une lotion, une émulsion eau-dans-huile (E/H), une émulsion huile-dans-eau (H/E) ou encore une émulsion triple telle que eau-dans-huile-dans-eau (E/H/E) ou huile-dans-eau-dans-huile (H/E/H), la quantité de poudre incorporée va de préférence de 1 à 50 % et mieux de 10 à 40 % en poids par rapport au poids total de la composition. On entend ici par "émulsion" aussi bien des dispersions sans émulsionnants que des dispersions comportant des émulsionnants ou encore des dispersions stabilisées par des sphérules lipidiques de type ionique ou non ionique.

La quantité d'électrolyte(s) dans une telle composition peut aller par exemple de 2 à 15 % et de préférence de 3 à 8 % en poids par rapport au poids total de la composition.

Ces compositions sont destinées à une application topique et comprennent de manière appropriée un milieu physiologiquement acceptable pour la peau, les muqueuses et/ou les fibres kératiniques (cheveux et cils).

Lorsque la composition est une émulsion, cette dernière contient de façon classique au moins une huile et éventuellement un émulsionnant approprié.

La nature de l'émulsionnant ou des émulsionnants utilisés dépend du type d'émulsion que l'on souhaite obtenir. Comme émulsionnants, on peut citer par exemple les esters d'acide gras et de polyols oxyéthylénés ou non oxyéthylénés, tels que le stéarate de sucrose et le stéarate de sorbitan, Le ou les émulsionnants peuvent être présents dans la composition selon l'invention, dans une concentration qui peut varier dans une large mesure. Ainsi, cette concentration peut aller par exemple de 0,1 à 20 %, et de préférence de 1 à 5 % du poids total de la composition.

La nature de la phase huileuse rentrant dans la composition des émulsions n'est pas critique et elle peut ainsi être constituée par tous les corps gras classiquement utilisés dans les domaines cosmétique et dermatologique et notamment les huiles indiquées ci-dessus. La phase huileuse de l'émulsion peut représenter de 1 à 50 % et mieux de 5 à 40 % en poids du poids total de l'émulsion.

En outre, de façon connue, les compositions de l'invention peuvent contenir des adjuvants habituels dans les domaines cosmétique et dermatologique, tels que les actifs hydrophiles ou lipophiles, les conservateurs, les antioxydants, les parfums, les charges, les matières colorantes (pigments ou colorants), les filtres solaires, et encore les vésicules lipidiques. Ces adjuvants sont utilisés dans les proportions habituelles dans le domaine cosmétique ou dermatologique, et par exemple de 0,01 à 20 % du poids total de l'émulsion, et ils sont, selon leur nature, introduits dans la phase aqueuse ou dans la phase huileuse de la composition, ou encore dans des vésicules.

Selon la fluidité de la composition que l'on souhaite obtenir, on peut y ajouter un ou plusieurs gélifiants comme les argiles, les gommes polysaccharides et leurs dérivés (gomme de xanthane, carboxyméthyl-hydroxypropyl guar), les polymères carboxyvinyliques ou carbomers tels que les produits commercialisés sous la dénomination Carbopol par la société Goodrich. Ces gélifiants sont généralement utilisés à des concentrations allant de 0,1 à 10 %, de préférence 0,1 à 5 % et mieux de 0,1 à 3 % du poids total de la composition.

Les quantités des différents constituants des compositions selon l'invention sont celles classiquement utilisées dans les domaines considérés. La nature des adjuvants et leurs quantités doivent être telles qu'elles ne modifient pas les propriétés des compositions selon l'invention.

L'utilisation ultérieure de la poudre de l'invention ou de la composition la contenant dépend du but recherché et des actifs éventuellement présents. La poudre selon l'invention ou la composition contenant une telle poudre peuvent notamment être utilisées pour le traitement et le soin de la peau, des muqueuses, du cuir chevelu et/ou des cheveux, en particulier pour le traitement de la peau sensible et du cuir chevelu sensible et/ou pour hydrater la peau, ainsi que pour le traitement des désordres cutanés tels que la peau sèche, le psoriasis, les dermites atopiques, le vitiligo, et le mycosis fongoïde.

Aussi, la présente invention a encore pour objet l'utilisation cosmétique de la poudre ou de la composition telles que définies ci-dessus, pour traiter la peau sensible et/ou le cuir chevelu sensible et/ou pour hydrater la peau. Pour de plus amples détails concernant la peau sensible, on peut se référer au document EP-A-680749.

La présente invention a aussi pour objet l'utilisation de la poudre ou de la composition telles que définies ci-dessus pour la fabrication d'une composition dermatologique destinée à traiter les désordres cutanés, et notamment la peau sèche, le psoriasis, les dermites atopiques, le vitiligo, et le mycosis fongoïde.

Les exemples ci-après de composition selon l'invention sont donnés à titre d'illustration et sans caractère limitatif. Les quantités y sont données en % en poids.

### Exemple 1: Poudre de soin de la peau

- Amidon modifié (DRY FLO) 18,2 %
- Huile d'abricot 60,6 %
- Sels de la mer morte (avec 37,5 % d'eau de recristallisation) 21,2 %

Mode opératoire : on prépare un mélange contenant 6 % d'amidon modifié, 7 % de sels et 67 % d'eau et on chauffe le mélange à 95°C, puis on le refroidit à 70 C avant d'incorporer 20 % d'huile d'abricot, tout en maintenant une agitation constante de manière à obtenir une dispersion huile-dans-eau. On refroidit la dispersion jusqu'à température ambiante tout en l'agitant et on l'homogénéise sous une pression d'environ 600 bars (60 x 10⁶ Pa), puis on la passe dans un appareil d'atomisation où l'air chaud est à 150°C et la température de sortie est de 80°C.

La poudre peut être utilisée telle quelle, non reconstituée, et constitue un produit efficace pour des peaux sensibles, irritées et à tendance grasse. De plus cette poudre a l'avantage de contenir un fort pourcentage de sels tout en gardant un aspect et un toucher cosmétique malgré ces concentrations.

### Exemple 2 : Emulsion H/E

### Phase aqueuse:

- Stéarate de sucrose 3,6 %
- Glycérine 3 %
- Conservateur 0,25 %
- Eau 43,5 %

### Phase grasse

- Stéarate de sorbitan 2,4 %
- Fraction liquide de beurre de karité 5 %
- Huile de silicone volatile 12 %
- Gomme de xanthane 0.2 %
- Carbopol 0,05 %

### Ajustement pH

- Soude qsp pH 5,8

### Incorporation d'actif

- Poudre selon l'exemple 1 30 %

Mode opératoire : on fait chauffer la phase aqueuse et la phase grasse séparément à 80°C. Puis, on verse la phase grasse dans la phase aqueuse sous forte agitation et ensuite, on ajuste le pH avec la soude. Après avoir laissé refroidir à 40°C environ, on ajoute sous agitation plus faible la poudre selon l'exemple 1.

On obtient une émulsion stable, agréable à utiliser et apte à traiter les peaux sensibles.

## Revendications

1. Poudre cosmétique et/ou dermatologique, caractérisée en ce qu'elle comprend (i) de 5 à 60 % en poids, par rapport au poids total de la poudre, d'au moins un amidon modifié par au moins une réaction choisie parmi la prégélatinisation, l'oxydation, la réticulation et l'estérification, (ii) de 30 à 90 % en poids, par rapport au poids total de la poudre, d'une phase huileuse comprenant au moins une huile, et (iii) au moins un électrolyte, le rapport pondéral phase huileuse/amidon étant égal ou supérieur à 1.

2. Poudre selon la revendication 1, caractérisée en ce qu'elle est exempte de protéine.

3. Poudre selon la revendication 1 ou 2, caractérisée en ce qu'elle comprend de 5 à 50 % en poids d'au moins un amidon modifié, de 0,1 à 40 % en poids d'au moins un électrolyte et de 50 à 90 % en poids d'une phase huileuse comprenant au moins une huile, par rapport au poids total de la poudre.

4. Poudre selon l'une quelconque des revendications précédentes, caractérisée en ce que l'amidon modifié est l'amidon estérifié par l'anhydride octénylsuccinique.

5. Poudre selon l'une quelconque des revendications précédentes, caractérisée en ce que la phase huileuse comprend au moins une huile choisie parmi les huiles minérales, les huiles de silicone, les huiles d'origine végétale, les huiles d'origine animale et les huiles de synthèse.

6. Poudre selon l'une quelconque des revendications précédentes, caractérisée en ce que la phase huileuse contient en outre au moins une matière grasse choisie parmi les acides gras, les alcools gras et les cires.

7. Poudre selon l'une quelconque des revendications précédentes, caractérisée en ce que la quantité d'électrolyte(s) va de 0,1 à 40 % en poids par rapport au poids total de la poudre.

8. Poudre selon l'une quelconque des revendications précédentes, caractérisée en ce que l'électrolyte est un sel de métal mono-, di- ou trivalent.

9. Poudre selon la revendication précédente, caractérisée en ce que le sel est choisi parmi les sels de baryum, de calcium, de strontium, de sodium, de potassium, de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.

10. Poudre selon l'une quelconque des revendications précédentes, caractérisée en ce que l'électrolyte est constitué d'ions choisis parmi les ions chlorure, borate, bicarbonate, carbonate, nitrate, hydroxyde, sulfate, persulfate, glycérophosphate, acétate, d'α-hydroxyacides, d'acides de fruit, d'acides aminés.

11. Poudre selon l'une quelconque des revendications précédentes, caractérisée en ce que l'électrolyte est choisi parmi le nitrate de calcium, de magnésium ou de strontium, le borate de calcium ou de magnésium, le chlorure de calcium, de magnésium, de sodium, de strontium, de néodyme ou de manganèse, le sulfate de magnésium ou de calcium, l'acétate de calcium ou de magnésium, et leurs mélanges.

12. Poudre selon l'une quelconque des revendications précédentes, caractérisée en ce qu'elle contient en outre, au moins un additif choisi parmi les actifs cosmétiques ou dermatologiques, les émulsionnants, les détergents, les matières colorantes, les abrasifs, les agents antioxydants ou anti-radicaux libres, les parfums et les charges.

13. Procédé de fabrication d'une poudre cosmétique et/ou dermatologique, comprenant au moins un amidon modifié, au moins un électrolyte et une phase huileuse, comprenant (1) la préparation d'une dispersion huile-dans-eau par mélange d'une phase huileuse comprenant au moins une huile dans une phase aqueuse comprenant au moins un amidon modifié et au moins un électrolyte, le rapport pondéral phase huileuse/amidon étant égal ou supérieur à 1, et (2) la déshydratation de ladite dispersion pour obtenir ladite poudre.

14. Procédé selon la revendication 13, caractérisé en ce que la phase aqueuse de la dispersion huile-dans-eau représente au moins 30% du poids total de la dispersion.

15. Procédé selon la revendication 13 ou 14, caractérisé en ce qu'il comprend une étape d'homogénéisation entre la préparation de la dispersion et avant la déshydratation de ladite dispersion.

16. Procédé selon la revendication précédente, caractérisé en ce que l'homogénéisation est réalisée sous une pression d'environ 600 bars.

17. Procédé selon l'une quelconque des revendications 13 à 16, caractérisé en ce que la déshydratation est effectuée par atomisation.

18. Procédé selon la revendication précédente, caractérisé en ce que la déshydratation est réalisée dans un appareil d'atomisation où la température de l'air chaud utilisé pour le séchage va d'environ 100°C à 220°C, et la température de sortie de la poudre va d'environ 30°C à 140°C.

19. Poudre cosmétique et/ou dermatologique susceptible d'être obtenue par le procédé selon l'une quelconque des revendications 13 à 18.

20. Composition cosmétique et/ou dermatologique, caractérisée en ce qu'elle comprend au moins une poudre selon l'une quelconque des revendications 1 à 12 ou une poudre selon la revendication 19.

21. Composition selon la revendication précédente, caractérisée en ce qu'elle comporte au moins une phase aqueuse.

22. Composition selon la revendication 20 ou 21, caractérisée en ce que la quantité de poudre va de 1 à 40 % en poids par rapport au poids total de la composition.

23. Composition selon l'une quelconque des revendications 20 à 22, caractérisée en ce qu'elle contient de 2 à 15 % en poids d'électrolyte(s) par rapport au poids total de la composition.

24. Composition selon l'une quelconque des revendications 20 à 23, caractérisée en ce qu'elle se présente sous forme d'une lotion, d'une émulsion eau-dans-huile, d'une émulsion huile-dans-eau ou d'une émulsion triple.

25. Utilisation cosmétique de la poudre selon l'une quelconque des revendications 1 à 12 et 19 ou de la composition selon l'une quelconque des revendications 20 à 24, pour traiter la peau sensible et/ou le cuir chevelu sensible et/ou pour hydrater la peau.

26. Utilisation de la poudre selon l'une quelconque des revendications 1 à 12 et 19 ou de la composition selon l'une quelconque des revendications 20 à 25, pour la préparation d'une composition dermatologique destinée à traiter les désordres cutanés.
